(19) Europäisches Patentamt
European Patent Office
Office européen des brevets

(11) Veröffentlichungsnummer: **0 077 479**
**A2**

(12)
# EUROPÄISCHE PATENTANMELDUNG

(21) Anmeldenummer: 82108991.9

(22) Anmeldetag: 29.09.82

(51) Int. Cl.³: C 07 D 233/60
C 07 D 233/54, C 07 D 249/08
A 01 N 43/50, A 01 N 43/64
//C07C49/84

(30) Priorität: 10.10.81 DE 3140276

(43) Veröffentlichungstag der Anmeldung:
27.04.83 Patentblatt 83/17

(84) Benannte Vertragsstaaten:
AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Elbe, Hans-Ludwig, Dr.
Dasnöckel 59
D-5600 Wuppertal 11(DE)

(72) Erfinder: Büchel, Karl Heinz, Prof. Dr.
Dabringhausener Strasse 42
D-5093 Burscheid(DE)

(72) Erfinder: Brandes, Wilhelm, Dr.
Eichendorffstrasse 3
D-5653 Leichlingen 1(DE)

(72) Erfinder: Frohberger, Paul-Ernst, Dr.
Willi-Baumeister-Strasse 5
D-5090 Leverkusen 1(DE)

(72) Erfinder: Paul, Volker, Dr.
Ahornstrasse 5
D-5650 Solingen 1(DE)

(54) **Phenoxyphenyl-azolylmethyl-ketone und -carbinole, Verfahren zu ihrer Herstellung und ihre Verwendung als Fungizide und als Zwischenprodukte.**

(57) Die Erfindung betrifft neue Phenoxyphenyl-azolylmethylketone und -carbinole, mehrere Verfahren zu ihrer Herstellung sowie ihre Verwendung als Fungizide und als Zwischenprodukte für die Synthese von weiteren Pflanzenschutzmitteln.

Die Verbindungen der allgemeinen Formel

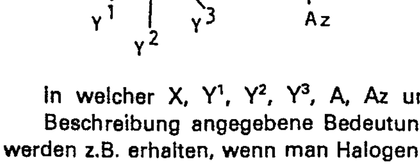

(1)

in welcher X, $Y^1$, $Y^2$, $Y^3$, A, Az und $R^1$ die in der Beschreibung angegebene Bedeutung besitzen,
werden z.B. erhalten, wenn man Halogenketone mit Azolen umsetzt und gegebenenfalls die erhaltenen Azolylketone alkyliert und gegebenenfalls noch mit Hydriden reduziert oder mit metallorganischen Verbindungen umsetzt.

Die Wirkstoffe sind für den Gebrauch als Pflanzenschutzmittel geeignet und können insbesondere zur Bekämpfung solcher Pilze eingesetzt werden, die echte Mehltauerkrankungen hervorrufen.

EP 0 077 479 A2

0077479

BAYER AKTIENGESELLSCHAFT 5090 Leverkusen, Bayerwerk
Zentralbereich Slr/Fr
Patente, Marken und Lizenzen Ib

Ib

# Phenoxyphenyl-azolylmethyl-ketone und -carbinole, Verfahren zu ihrer Herstellung und ihre Verwendung als Fungizide und als Zwischenprodukte

Die vorliegende Erfindung betrifft neue Phenoxyphenyl-azolylmethyl-ketone und -carbinole, mehrere Verfahren zu ihrer Herstellung sowie ihre Verwendung als Fungizide und als Zwischenprodukte für die Synthese von weiteren Pflanzenschutzmitteln.

Es ist bereits bekannt geworden, daß Phenyl-azolylmethylketone und -carbinole fungizide Eigenschaften besitzen (vergleiche DE-OS 24 31 407). Die Wirkung dieser Azolyl-Derivate ist jedoch in vielen Indikationsbereichen, insbesondere bei niedrigen Aufwandmengen und -konzentrationen, nicht immer ganz befriedigend.

Es wurden neue Phenoxyphenyl-azolylmethyl-ketone und -carbinole der allgemeinen Formel

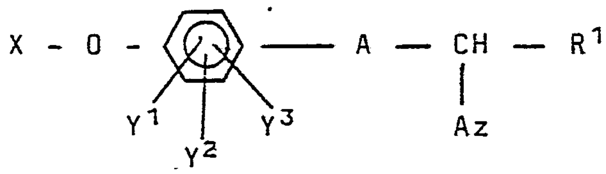

(I)

in welcher

Az für 1,2,4-Triazol-1-yl und -4-yl oder Imidazol-1-yl steht,

A für die Ketogruppe, die -CH(OH)- oder die -C(OH)R-Gruppierung steht,.

R für Alkyl, Cycloalkyl, Cycloalkylalkyl, Alkenyl, Alkinyl, gegebenenfalls substituiertes Phenyl oder gegebenenfalls substituiertes Phenalkyl steht,

$R^1$ für Alkyl, Cycloalkyl, Cycloalkylalkyl, Alkenyl, Alkinyl, Halogenalkyl, Alkoxyalkyl, Halogenalkoxyalkyl, Alkylthioalkyl, Halogenalkylthioalkyl, Dialkylaminoalkyl oder gegebenenfalls substituiertes Phenalkyl steht, sowie auch für Wasserstoff steht, wenn nicht gleichzeitig X für unsubstituiertes Phenyl und $Y^1$, $Y^2$ und $Y^3$ für Wasserstoff stehen, und ferner noch für Wasserstoff steht, wenn R nicht für Alkyl, Cycloalkyl oder Cycloalkylalkyl steht,

X für gegebenenfalls einfach oder mehrfach, gleich oder verschieden substituiertes Phenyl steht,

$Y^1$, $Y^2$, $Y^3$ gleich oder verschieden sind und für Wasserstoff, Halogen oder Alkyl stehen,

sowie deren pflanzenverträglichen Säureadditions-Salze und Metallsalz-Komplexe gefunden.

Die Verbindungen der Formel (I) besitzen gegebenenfalls zwei asymmetrische Kohlenstoffatome; sie können dann in den beiden geometrischen Isomeren (threo- und erythro-Form) vorliegen, die in unterschiedlichen Mengenverhältnissen anfallen können. In beiden Fällen liegen sie als optische Isomeren vor.

Weiterhin wurde gefunden, daß man die Phenoxyphenyl-azolylmethyl-ketone und -carbinole der Formel (I) erhält, wenn man

a) Halogenketone der Formel

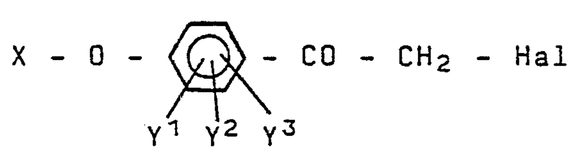

(II)

in welcher

Hal für Halogen, vorzugsweise Chlor oder Brom steht und

X, $Y^1$, $Y^2$ u. $Y^3$ die oben angegebene Bedeutung haben,

mit Azolen der Formel

H - Az (III)

in welcher

Az die oben angegebene Bedeutung hat,

in Gegenwart eines Verdünnungsmittels und in Gegenwart eines Säurebindemittels umsetzt; und gegebenenfalls

b) die so erhaltenen Azolylketone der Formel

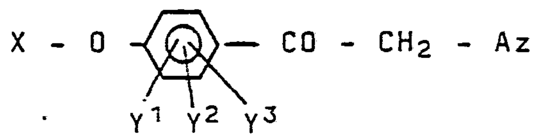 (Ia)

in welcher

Az, X, $Y^1$, $Y^2$ und $Y^3$ die oben angegebene Bedeutung haben,

mit einem Alkylierungsmittel der Formel

$$R^1 - Z \qquad (IV)$$

in welcher

$R^1$ die oben angegebene Bedeutung hat und

Z für eine elektronenanziehende Abgangsgruppierung steht,

in Gegenwart einer Base und in Gegenwart eines organischen Verdünnungsmittels, oder in einem wässrig-organischen Zweiphasensystem in Gegenwart eines Phasentransfer-Katalysators, umsetzt; und gegebenenfalls

c) die nach den Verfahren (a) und (b) erhaltenen Azolylketone der Formel

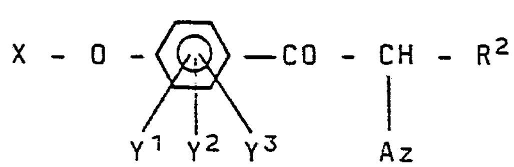 (Ib)

in welcher

Az, X, $Y^1$, $Y^2$ und $Y^3$ die oben angegebene Bedeutung haben und

$R^2$ für Wasserstoff oder $R^1$ steht, wobei $R^1$ die oben angegebene Bedeutung hat,

durch Reaktion mit komplexen Hydriden bzw. Aluminiumisopropylat in Gegenwart eines Verdünnungsmittels reduziert oder mit metallorganischen Verbindungen der Formel

Me - R (V)

in welcher

R die oben angegebene Bedeutung hat und

Me für ein Alkalimetall oder den Rest Hal'-Mg steht, wobei

Hal' für Chlor, Brom oder Iod steht,

in Gegenwart eines Verdünnungsmittels umsetzt.

An die so erhaltenen Verbindungen der Formel (I) kann gegebenenfalls anschließend eine Säure oder ein Metallsalz addiert werden. In manchen Fällen erweist es sich als vorteilhaft, die Verbindungen der Formel (I) über ihre Salze in reiner Form zu erhalten.

Die neuen Phenoxyphenyl-azolylmethyl-ketone und -carbinole der Formel (I) weisen starke fungizide Eigenschaften auf. Dabei zeigen überraschenderweise die erfindungsgemäßen Verbindungen eine bessere fungizide Wirkung als die aus dem Stand der Technik bekannten Phenyl-azolylmethylketone und -carbinole, welche chemisch und wirkungsmäßig ähnliche Verbindungen sind.

Außerdem sind die neuen Phenoxyphenyl-azolylmethyl-ketone und -carbinole der Formel (I) interessante Zwischenprodukte zur Herstellung von weiteren Pflanzenschutzmitteln. Bei den Keto-Derivaten kann die Ketogruppe zu einer -CH(OH)-Gruppe bzw. einer -CR(OH)-Gruppe reduziert werden (vgl.die Verfahrensvariante c). Ferner können durch entsprechende Umsetzungen funktionelle Derivate der Ketogruppe erhalten werden, wie z.B. Oxime und Oximether, Hydrazone und Ketale. Die Carbinol-Derivate können an der Hydroxy-Gruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der Formel (I) erhalten werden.

Die erfindungsgemäßen Stoffe stellen somit eine wertvolle Bereicherung der Technik dar.

Die erfindungsgemäßen Phenoxyphenyl-azolylmethyl-ketone und -carbinole sind durch die Formel (I) allgemein definiert. Bevorzugt sind Verbindungen der allgemeinen Formel

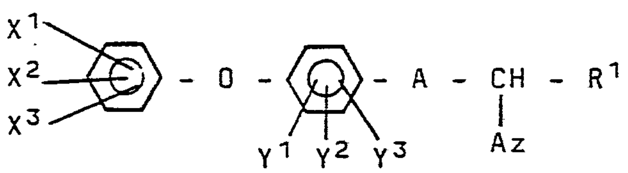 (IA)

in welcher

Az für 1,2,4-Triazol-1-yl und -4-yl oder Imidazol-1-yl steht,

A für die Ketogruppe, die -CH(OH)- oder die -C(OH)R-Gruppierung steht,

R für geradkettiges oder verzweigtes Alkyl mit 1 bis 12 Kohlenstoffatomen, Cycloalkyl mit

3 bis 7 Kohlenstoffatomen, Cycloalkylalkyl mit 3 bis 7 Kohlenstoffatomen im Cycloalkylteil und 1 bis 2 Kohlenstoffatomen im Alkylteil, Alkenyl und Alkinyl mit jeweils 2 bis 6 Kohlenstoffatomen, gegebenenfalls substituiertes Phenyl und gegebenenfalls substituiertes Phenalkyl mit 1 bis 4 Kohlenstoffatomen im Alkylteil steht, wobei als Phenylsubstituenten beispielsweise genannt seien: Halogen und Alkyl mit 1 bis 4 Kohlenstoffatomen,

$R^1$ für geradkettiges oder verzweigtes Alkyl mit 1 bis 12 Kohlenstoffatomen, Cycloalkyl mit 3 bis 7 Kohlenstoffatomen, Cycloalkylalkyl mit 3 bis 7 Kohlenstoffatomen im Cycloalkylteil und 1 bis 2 Kohlenstoffatomen im Alkylteil, Alkenyl und Alkinyl mit jeweils 2 bis 6 Kohlenstoffatomen, Alkoxyalkyl und Alkylthioalkyl mit jeweils 1 bis 2 Kohlenstoffatomen in jedem Alkylteil, Halogenalkyl mit 1 bis 8 Kohlenstoffatomen und 1 bis 5 Halogenatomen, wie vorzugsweise Fluor- und Chloratomen, Halogenalkoxyalkyl und Halogenalkylthioalkyl mit jeweils 1 bis 4 Kohlenstoffatomen in jedem Alkylteil und 1 bis 5 Halogenatomen, wie vorzugsweise Fluor- und Chloratomen, Dialkylaminoalkyl mit 1 bis 4 Kohlenstoffatomen in jedem Alkylteil, und gegebenenfalls substituiertes Phenylalkyl mit 1 bis 4 Kohlenstoffatomen im Alkylteil steht, wobei als Phenylsubstituenten die für $X^1$ genannten Reste infrage kommen , sowie auch für Wasserstoff steht, wenn nicht gleichzeitig $X^1$,$X^2$,$X^3$,$Y^1$,$Y^2$ und $Y^3$ für Wasserstoff stehen, und ferner noch für Wasserstoff steht, wenn R nicht für Alkyl, Cycloalkyl oder Cycloalkylalkyl steht,

0077479

$X^1$ für Wasserstoff, Halogen, Alkyl mit 1 bis 4 Kohlenstoffatomen; Halogenalkyl, Halogenalkoxy und Halogenalkylthio mit jeweils 1 bis 4 Kohlenstoffatomen und 1 bis 5 Halogenatomen, wie vorzugsweise Fluor- und Chloratomen, Alkoxy und Alkythio mit jeweils 1 bis 4 Kohlenstoffatomen, Amino, Alkylamino und Dialkylamino mit jeweils 1 bis 4 Kohlenstoffatomen in jedem Alkylteil, Alkoxycarbonyl mit 1 bis 4 Kohlenstoffatomen im Alkylteil, Nitro oder Cyano steht,

$X^2$ für Wasserstoff, Halogen, Alkyl und Alkoxy mit jeweils 1 bis 4 Kohlenstoffatomen steht,

$X^1$ und $X^2$ gemeinsam in o-Stellung zueinander für Methylendioxo stehen,

$X^3$ für Wasserstoff, Halogen, Alkyl und Alkoxy mit jeweils 1 bis 4 Kohlenstoffatomen steht,

$Y^1$ für Wasserstoff, Halogen und Alkyl mit 1 bis 4 Kohlenstoffatomen steht,

$Y^2$ für Wasserstoff oder Halogen steht und

$Y^3$ für Wasserstoff steht.

Besonders bevorzugt sind diejenigen Verbindungen der Formel (IA), in denen

A und Az die oben angegebene Bedeutung haben,

R für geradkettiges oder verzweigtes Alkyl mit 1 bis 4 Kohlenstoffatomen, Cycloalkyl und Cycloalkylmethyl mit jeweils 5 oder 6 Kohlenstoffatomen im Cycloalkylteil, Alkenyl und Alkinyl mit jeweils 2 bis 4 Kohlenstoffatomen, gegebenenfalls substituiertes Phenyl und Benzyl steht, wobei als Substituenten genannt seien: Fluor, Chlor, Brom und Methyl,

$R^1$ für geradkettiges oder verzweigtes Alkyl mit 1 bis 8 Kohlenstoffatomen, Cycloalkyl und Cycloalkylmethyl mit jeweils 5 oder 6 Kohlenstoffatomen im Cycloalkylteil, Alkenyl mit 2 bis 6 Kohlenstoffatomen, Alkinyl mit 2 bis 4 Kohlenstoffatomen, Alkoxyalkyl und Alkylthioalkyl mit jeweils 1 bis 2 Kohlenstoffatomen in jedem Alkylteil, Halogenalkyl mit 1 bis 4 Kohlenstoff- und 1 bis 5 Halogenatomen, wie insbesondere Fluor- und Chloratomen, Halogenalkoxyalkyl und Halogenalkylthioalkyl mit jeweils 1 bis 2 Kohlenstoffatomen in jedem Alkylteil und 1 bis 5 Halogenatomen, wie insbesondere Fluor- und Chloratomen, Dialkylaminoalkyl mit 1 bis 2 Kohlenstoffatomen in jedem Alkylteil, und gegebenenfalls substituiertes Benzyl steht, wobei als Phenylsubstituenten die für $X^1$ genannten Reste infrage kommen, sowie auch für Wasserstoff steht, wenn nicht gleichzeitig $X^1$, $X^2$, $X^3$, $Y^1$, $Y^2$ und $Y^3$ für Wasserstoff stehen, und ferner noch für Wasserstoff steht, wenn R nicht für Alkyl, Cycloalkyl oder Cycloalkylalkyl steht,

$X^1$ für Wasserstoff, Fluor, Chlor, Brom, Methyl, Trifluormethyl, Trifluormethoxy, Trifluormethylthio, Methoxy, Methylthio, Amino, Dimethylamino, Methoxycarbonyl, Nitro oder Cyano steht,

$X^2$ für Wasserstoff, Fluor, Chlor, Methyl oder Methoxy steht,

$X^1$ und $X^2$ gemeinsam in o-Stellung zueinander für Methylendioxo stehen,

$X^3$ für Wasserstoff, Fluor, Chlor, Methyl oder Methoxy steht,

$Y^1$ für Wasserstoff, Fluor, Chlor oder Methyl steht,

$Y^2$ für Wasserstoff, Fluor oder Chlor steht und

$Y^3$ für Wasserstoff steht.

Im einzelnen seien außer den bei den Herstellungsbeispielen genannten Verbindungen folgende Verbindungen der allgemeinen Formel (IA) genannt (wobei Az für 1,2,4-Triazol-1-yl oder Imidazol-1-yl steht):

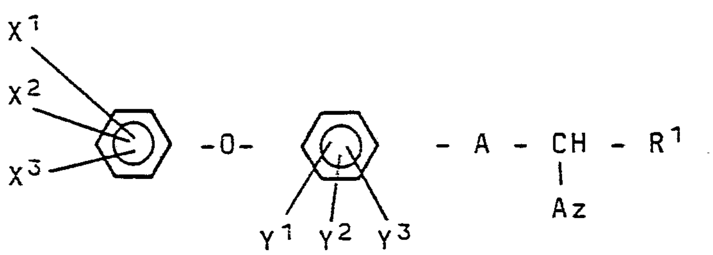

(IA)

| | $X^1$ | $X^2$ | $X^3$ | $Y^1$ | $Y^2$ | $Y^3$ | A | $R^1$ |
|---|---|---|---|---|---|---|---|---|
| 4-Cl | H | H | H | H | H | -C(OH)- 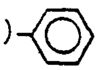 | H |
| 4-Cl | H | H | H | H | H | $-C(OH)-CH_2-$ 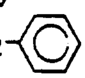 | H |
| 4-Cl | H | H | H | H | H | $-C(OH)-CH_2CH=CHCH_3$ | H |
| 4-Cl | H | H | H | H | H | $-C(OH)-C_4H_9$ | H |
| 4-Cl | H | H | H | H | H | $-C(OH)-CH_2-C\equiv CH$ | H |
| 4-F | H | H | H | H | H | -C(OH)- 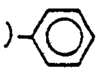 | H |
| 4-F | H | H | H | H | H | $-C(OH)-CH_2-$ 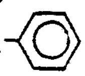 | H |
| 4-F | H | H | H | H | H | $-C(OH)-CH_2CH=CHCH_3$ | H |
| 4-F | H | H | H | H | H | $-C(OH)-C_4H_9$ | H |
| 4-F | H | H | H | H | H | $-C(OH)-CH_2-C\equiv CH$ | H |
| 4-F | H | H | H | H | H | -CO- | $CH_3$ |
| 4-F | H | H | H | H | H | -CO- | $C_2H_5$ |
| 4-F | H | H | H | H | H | -CO- | $C_3H_7$ |
| 4-F | H | H | H | H | H | -CO- | $C_4H_9$ |
| 4-F | H | H | H | H | H | -CO- | $C_5H_{11}$ |
| 4-F | H | H | H | H | H | -CO- | $C_3H_7$-i |
| 4-F | H | H | H | H | H | -CH(OH)- | $CH_3$ |
| 4-F | H | H | H | H | H | -CH(OH)- | $C_2H_5$ |
| 4-F | H | H | H | H | H | -CH(OH)- | $C_3H_7$ |
| 4-F | H | H | H | H | H | -CH(OH)- | $C_4H_9$ |
| 4-F | H | H | H | H | H | -CH(OH)- | $C_5H_{11}$ |
| 4-F | H | H | H | H | H | -CH(OH)- | $C_3H_7$-i |

| $X^1$ | $X^2$ | $X^3$ | $Y^1$ | $Y^2$ | $Y^3$ | A | $R^1$ |
|---|---|---|---|---|---|---|---|
| $4-N(CH_3)_2$ | H | H | H | H | H | -CO- | $C_3H_7$ |
| $-O-CH_2-O-$ | | H | H | H | H | -CO- | $C_3H_7$ |
| $4-CH_3$ | H | H | H | H | H | -CO- | $C_3H_7$ |
| $3-OCH_3$ | $4-OCH_3$ | $5-OCH_3$ | H | H | H | -CO- | $C_3H_7$ |
| $4-CO-OCH_3$ | H | H | H | H | H | -CO- | $C_3H_7$ |
| $4-SCF_3$ | H | H | H | H | H | -CO- | $C_3H_7$ |
| $4-NO_2$ | H | H | H | H | H | -CO- | $C_3H_7$ |
| 4-CN | H | H | H | H | H | -CO- | $C_3H_7$ |
| 4-Br | H | H | H | H | H | -CO- | $C_3H_7$ |
| $4-OCF_3$ | H | H | H | H | H | -CO- | $C_3H_7$ |
| $4-N(CH_3)_2$ | H | H | H | H | H | -CH(OH)- | $C_3H_7$ |
| $-O-CH_2-O-$ | | H | H | H | H | -CH(OH)- | $C_3H_7$ |
| $4-CH_3$ | H | H | H | H | H | -CH(OH)- | $C_3H_7$ |
| $3-OCH_3$ | $4-OCH_3$ | $5-OCH_3$ | H | H | H | -CH(OH)- | $C_3H_7$ |
| $4-CO-OCH_3$ | H | H | H | H | H | -CH(OH)- | $C_3H_7$ |
| $4-SCF_3$ | H | H | H | H | H | -CH(OH)- | $C_3H_7$ |
| $4-NO_2$ | H | H | H | H | H | -CH(OH)- | $C_3H_7$ |
| 4-CN | H | H | H | H | H | -CH(OH)- | $C_3H_7$ |
| 4-Br | H | H | H | H | H | -CH(OH)- | $C_3H_7$ |
| $4-OCF_3$ | H | H | H | H | H | -CH(OH)- | $C_3H_7$ |
| 4-F | H | H | H | H | H | -CO- | $-CH_2CH_2OCH_3$ |
| 4-F | H | H | H | H | H | -CO- | $-CH_2CH_2SCH_3$ |
| 4-F | H | H | H | H | H | -CO- | $-CH_2CH_2N(CH_3)_2$ |

| $X^1$ | $X^2$ | $X^3$ | $Y^1$ | $Y^2$ | $Y^3$ | A | $R^1$ |
|---|---|---|---|---|---|---|---|
| 4-F | H | H | H | H | H | -CH(OH)- | $-CH_2CH_2OCH_3$ |
| 4-F | H | H | H | H | H | -CH(OH)- | $-CH_2CH_2SCH_3$ |
| 4-F | H | H | H | H | H | -CH(OH)- | $-CH_2CH_2N(CH_3)_2$ |
| 4-Cl | H | H | H | H | H | -CH(OH)- | $-CH_2CH_2OCH_3$ |
| 4-Cl | H | H | H | H | H | -CH(OH)- | $-CH_2CH_2SCH_3$ |
| 4-Cl | H | H | H | H | H | -CH(OH)- | $-CH_2CH_2N(CH_3)_2$ |
| 4-Cl | H | H | H | H | H | -CO- | $-CH_2CH_2OCH_3$ |
| 4-Cl | H | H | H | H | H | -CO- | $-CH_2CH_2SCH_3$ |
| 4-Cl | H | H | H | H | H | -CO- | $-CH_2CH_2N(CH_3)_2$ |
| 4-Cl | H | H | 3-Cl | H | H | -CO- | $C_3H_7$ |
| 4-Cl | H | H | 3-Cl | H | H | -CH(OH)- | $C_3H_7$ |
| 4-Cl | H | H | $3-CH_3$ | H | H | -CO- | $C_3H_7$ |
| 4-Cl | H | H | $3-CH_3$ | H | H | -CH(OH)- | $C_3H_7$ |
| 4-Cl | H | H | H | H | H | $-C(OH)CH_3$ | $C_3H_7$ |
| 4-Cl | H | H | H | H | H | $-C(OH)-C_3H_7$ | $C_3H_7$ |
| 4-Cl | H | H | H | H | H | -CO- | $-CH_2-$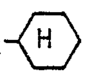 |
| 4-Cl | H | H | H | H | H | -CH(OH)- | $-CH_2-$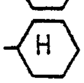 |

Verwendet man beispielsweise ω-Chlor-4-(4'-chlorphenoxy)-acetophenon und 1,2,4-Triazol als Ausgangsstoffe, so kann der Reaktionsablauf durch das folgende Formelschema wiedergegeben werden (Verfahren a):

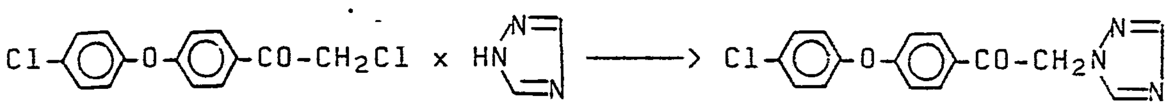

Verwendet man beispielsweise 4-(4'-Chlorphenoxy)-ω-(1,2,4-triazol-1-yl)-acetophenon und 4-Chlorbenzylchlorid als Ausgangsstoffe, so kann der Reaktionsablauf durch das folgende Formelschema wiedergegeben werden (Verfahren b):

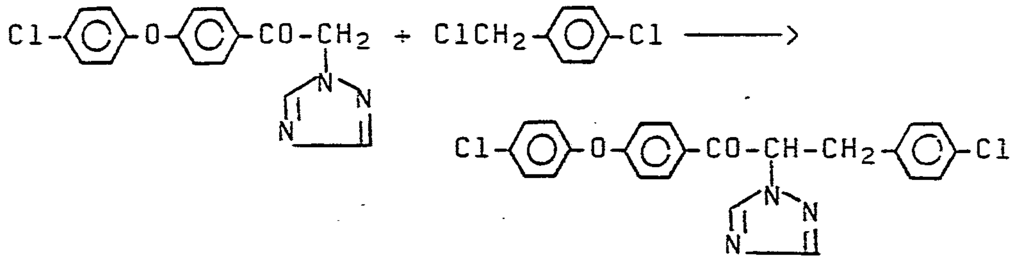

Verwendet man beispielsweise 4-(4'-Chlorphenoxy)-ω-(1,2,4-triazol-1-yl)-acetophenon und Natriumborhydrid als Ausgangsstoffe, so kann der Reaktionsablauf durch das folgende Formelschema wiedergegeben werden(Verfahren c):

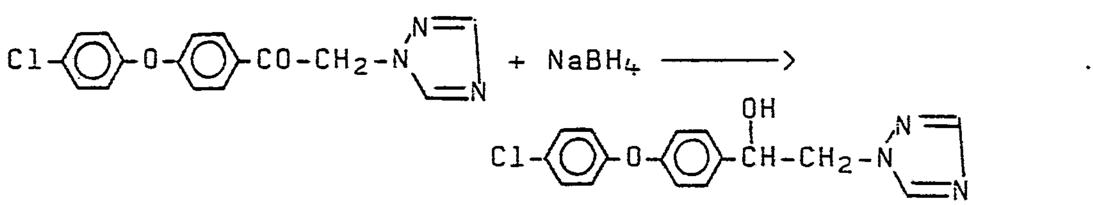

Verwendet man beispielsweise 1-(4-Chlorphenyl)-3-[4-(4'-chlorphenoxy)-phenyl]-2-(1,2,4-triazol-1-yl)-propan-3-on und Methylmagnesiumbromid als Ausgangsstoffe, so kann der Reaktionsablauf durch das folgende Formelschema wiedergegeben werden(Verfahren c):

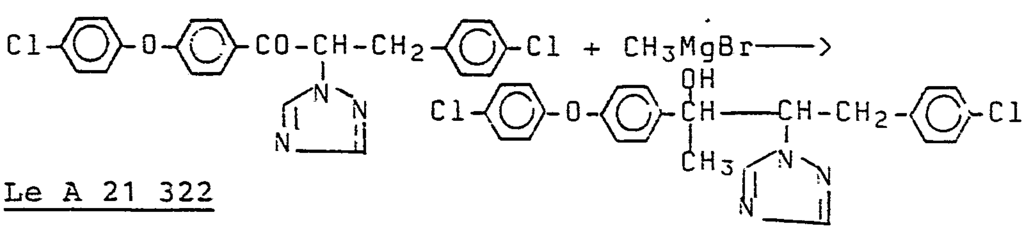

Die für die Durchführung des erfindungsgemäßen Verfahrens (a) als Ausgangsstoffe benötigten Halogenketone sind durch die Formel (II) allgemein definiert. In dieser Formel stehen X, $Y^1$, $Y^2$ und $Y^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenketone der Formel (II) sind noch nicht bekannt; sie können jedoch in allgemein bekannter Art und Weise erhalten werden, indem man z.B. Diphenylether der Formel

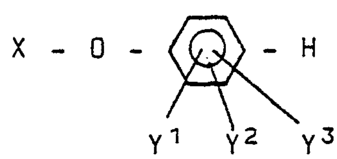

(VI)

in welcher

X, $Y^1$, $Y^2$ und $Y^3$ die oben angegebene Bedeutung haben,

mit Chlor(Brom)acetylchlorid(bromid) unter den Bedingungen einer Friedel-Crafts-Acylierung umsetzt (vergleiche auch die Herstellungsbeispiele).

Die außerdem für das erfindungsgemäße Verfahren (a) als Ausgangsstoffe zu verwendenden Azole sind durch die Formel (III) allgemein definiert. In dieser Formel steht Az für die in der Erfindungsdefinition angegebenen Bedeutungen.

Die Azole der Formel (III) sind allgemein bekannte Verbindungen der organischen Chemie.

Die für die Durchführung des erfindungsgemäßen Verfahrens (b) als Ausgangsstoffe zu verwendenden Azolylketone sind

durch die Formel (Ia) allgemein definiert. Die Verbindungen der Formel (Ia) sind erfindungsgemäße Stoffe.

Die außerdem für das erfindungsgemäße Verfahren (b) als Ausgangsstoffe zu verwendenden Alkylierungsmittel sind durch die Formel (IV) allgemein definiert. In dieser Formel steht $R^1$ vorzugsweise für diejenigen Reste, die bereits im Zusammenhang mit der Beschreibung der erfindungsgemäßen Stoffe der Formel (I) für diesen Substituenten vorzugsweise genannt wurden. Z steht vorzugsweise für eine elektronenanziehende Abgangsgruppierung, wie beispielsweise Halogen, p-Methylphenylsulfonyloxy, die Gruppierung $-O-SO_2-OR'$ oder $-NR'_3$ und andere, wobei R' z.B. für Alkyl mit 1 bis 4 Kohlenstoffatomen steht.

Die Alkylierungsmittel der Formel (IV) sind allgemein bekannte Verbindungen der organischen Chemie.

Die für die Durchführung des erfindungsgemäßen Verfahrens (c) als Ausgangsstoffe zu verwendenden Azolylketone sind durch die Formel (Ib) allgemein definiert. Die Verbindungen der Formel (Ib) sind erfindungsgemäße Stoffe.

Die unter anderem außerdem für das erfindungsgemäße Verfahren (c) als Ausgangsstoffe zu verwendenden metallorganischen Verbindungen sind durch die Formel (V) allgemein definiert. In dieser Formel steht R vorzugsweise für diejenigen Reste, die bereits im Zusammenhang mit der Beschreibung der erfindungsgemäßen Stoffe der Formel (I) vorzugsweise für diesen Substituenten genannt wurden. Me steht vorzugsweise für Lithium, Natrium und die sogenannte Grignard-Gruppierung Hal'-Mg, wobei Hal' für Chlor, Brom oder Iod steht.

Die metallorganischen Verbindungen der Formel (V) sind allgemein bekannte Verbindungen der organischen Chemie.

Für das erfindungsgemäße Verfahren (a) kommen als Verdünnungsmittel inerte organische Lösungsmittel infrage. Hierzu gehören vorzugsweise

Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol, oder Chlorbenzol; Formamide, wie insbesondere Dimethylformamid; und halogenierte Kohlenwasserstoffe.

Das erfindungsgemäße Verfahren (a) wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan.
Vorzugsweise verwendet man einen entsprechenden Ueberschuß an Azol.
Die Reaktionstemperaturen können bei dem erfindungsgemäßen Verfahren (a) in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) setzt man auf 1 Mol der Verbindungen der Formel (II) vorzugsweise 2 bis 4 Mol Azol und 1 bis 4 Mol Säurebinder ein. Zur Isolierung der Verbindungen der Formel (I)wird das Lösungsmittel abdestilliert und der Rückstand in üblicher Art und Weise aufgearbeitet.

Für das erfindungsgemäße Verfahren (b) kommen als Verdünnungsmittel inerte organische Lösungsmittel infrage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Das erfindungsgemäße Verfahren (b) wird in Gegegnwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens (b) in einem größeren Bereich variiert werden. Im allgemeinen arbeitet man zwischen 0 und 100°C, vorzugsweise zwischen 20 und 100°C.

Bei der Durchführung des erfindungsgemäßen Verfahrens (b) setzt man vorzugsweise auf 1 Mol der Verbindung der Formel (Ia) 1 bis 1,2 Mol Alkylierungsmittel ein. Die Isolierung der Endprodukte der Formel (I) erfolgt in allgemein üblicher Art und Weise.

Das erfindungsgemäße Verfahren (b) kann auch in einem Zweiphasensytem, wie beispielsweise wässrige Natron- oder Kalilauge/Toluol oder Methylenchlorid, gegebenenfalls unter Zusatz von 0,1 bis 1 Mol eines Phasen-Transfer-Katalysators, wie beispielsweise Ammonium- oder Phosphoniumverbindungen, beispielsweise seien Benzyldodecyl-dimethylammoniumchlorid und Triethyl-benzyl-ammoniumchlorid genannt, durchgeführt werden.

Die erfindungsgemäße Reduktion gemäß Verfahren (c) erfolgt in üblicher Weise.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wei Methanol, Ethanol, Butanol, Isopropanol und Ether, wei Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ib) etwa 1 Reaktionsäquivalent eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in eienm größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (Ib) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Arbeitet man mit metallorganischen Verbindungen, so kommen als Verdünnunsgmittel vorzugsweise wasserfreie Ether, wie Diethylether, Dibutylether oder Tetrahydrofuran, infrage. Die Reaktion wird allgemein bei 0 bis 80°C, vorzugsweise bei der Siedetemperatur des verwendeten Lösungsmittels durchgeführt. Hierzu setzt man auf 1 Mol der Verbindungen der Formel (Ib) etwa 1 bis 3 Mol der metallorgaischen Verbindung der Formel (V) ein. Die Aufarbeitung der durch metall-organische Reaktionen erhaltenen Gemische erfolgt in üblicher und allgemein bekannter Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I.bis II. sowie IV.bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) könnnen in einfacher Weise nach üblichen Verfahren erhalten werden, so z.B. durch Lösen des Metallsalzes in Alkohol, z.B. Ethanol und Hinzufügen zur Verbindungen der Formel(I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. des Getreidemehltaus (Erysiphe graminis), zur Bekämpfung von Venturia-Arten, wie z.B. gegen den Erreger des Apfelschorfs (Venturia inaequalis), oder zur Bekämpfung von Reiskrankheiten, wie z.B. Pyricularia oryzae und Pellicularia sasakii. Hervorzuheben ist, daß die erfindungsgemäßen Stoffe auch gute bakterizide Eigenschaften aufweisen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Fomulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

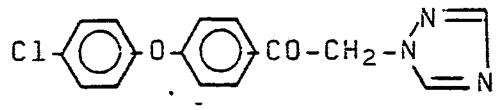

(Verfahren a)

100g (0,36 Mol) ω-Chlor-4-(4'-chlorphenoxy)-acetophenon, 31,1 g (0,45 Mol) 1,2,4-Triazol und 124 g (0,9 Mol) gemahlenes Kaliumcarbonat werden 20 Stunden unter Rückfluß und Rühren erhitzt. Danach läßt man abkühlen, saugt die anorganischen Salze ab und engt das Filtrat ein. Der Rückstand wird in Methylenchlorid verrührt und mit verdünnter Salzsäure versetzt. Das entstehende Hydrochlorid wird abgetrennt, mit Methylenchlorid gewaschen und in üblicher Weise durch Versetzen mit Alkalien und anschließendes Extrahieren in die freie Base überführt. Man erhält 41,5g (36,8% der Theorie) 4-(4'-Chlorphenoxy)-ω-(1,2,4-triazol-1-yl)-acetophenon vom Schmelzpunkt 148-150°C.

Herstellung des Ausgangsproduktes

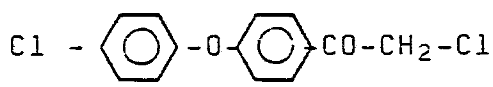

Zu 150g (0,73 Mol) 4-Chlorbiphenylether und 117g (0,88 Mol) Aluminiumtrichlorid in 1000 ml Methylenchlorid läßt man bei Raumtemperatur 86,6g (0,77 Mol) Chloracetylchlorid zutropfen. Die Reaktionslösung wird 1 Stunde nachgerührt und dann auf 3 l Eiswasser gegeben. Die organische Phase wird abgetrennt, mit Wasser gewaschen, über Natriumsulfat getrocknet und eingeengt. Der Rückstand wird mit Petrolether verrieben, abgesaugt und getrocknet. Man erhält 168,7g (82,3 % der Theorie) ω-Chlor-4-(4'-chlorphenoxy)-acetophenon vom Schmelzpunkt 59-61°C.

Beispiel 2

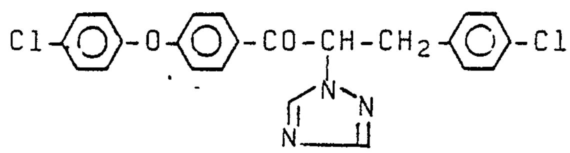

(Verfahren b)

31,4g (0,1 Mol) 4-(4'-Chlorphenoxy)-ω-(1,2,4-triazol-1-yl)-acetophenon (Beispiel 1), 16,1g 4-Chlorbenzylchlorid und 5,6g (0,1 Mol) Kaliumhydroxid werden in 200 ml Dimethylsulfoxid 20 Stunden bei 40°C gerührt. Anschließend wird das Reaktionsgemisch auf 700 ml Wasser gegeben und mit Essigester extrahiert. Die organische Phase wird mit Wasser gewaschen, über Natriumsulfat getrocknet und eingeengt. Der Rückstand wird zur Reinigung mit Isopropylether ausgekocht. Man erhält 26,1g (59,6 % der Theorie) 1-(4-Chlorphenyl)-3-[4-(4'-chlorphenoxy)-phenyl]-2-(1,2,4-triazol-1-yl)-propan-3-on vom Schmelzpunkt 132°C.

Beispiel 3

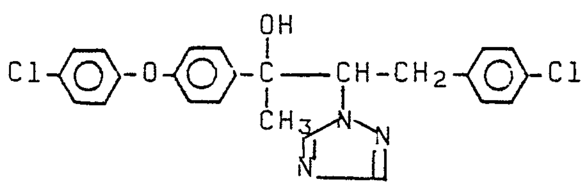

(Verfahren c)

7 g (0,016 Mol) 1-(4-Chlorphenyl)-3-[4-(4'-chlorphenoxy)-phenyl]-2-(1,2,4-triazol-1-yl)-propan-3-on (Beispiel 2) und 4,8g (0,04 Mol) Methylmagnesiumbromid, gelöst in 50 ml Ether, werden in 100 ml Tetrahydrofuran 1 Stunde unter Rückfluß gerührt. Man läßt abkühlen und versetzt mit verdünnter Salzsäure. Die organische Phase wird abgetrennt, mit Wasser gewaschen, über Natriumsulfat getrocknet und eingeengt. Der Rückstand wird in Diisopropylether verrührt,

abgesaugt und getrocknet. Man erhält 5,3g (73 % der Theorie) 1-(4-Chlorphenyl)-3-[4-(4'-chlorphenoxy)-phenyl]-2-(1,2,4-triazol-1-yl)-butan-3-ol vom Schmelzpunkt 159°C.

Beispiel 4

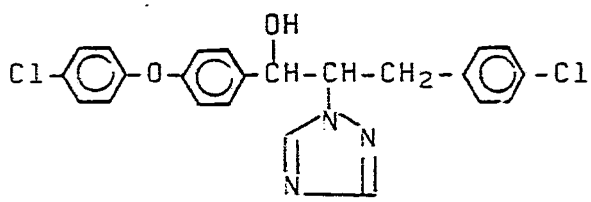

(Verfahren c)

14g (0,032 Mol) 1-(4-Chlorphenyl)-3-[4-(4'-chlorphenoxy)-phenyl]-2-(1,2,4-triazol-1-yl)-propan-3-on (Beispiel 2) in 400 ml Methanol werden mit 0,38 g (0,01 Mol) Natrium-borhydrid, gelöst in 10 ml Wasser, versetzt. Man läßt 2 Stunden bei 25°C rühren und stellt dann durch Zugabe von verdünnter Salzsäure auf einen pH-Wert von 6-7 ein. Die Reaktionslösung wird eingeengt und der Rückstand mit Methylenchlorid /Wasser aufgenommen. Man trennt die organische Phase ab, trocknet über Natriumsulfat und engt ein. Der feste Rückstand wird aus Diisopropylether umkristallisiert. Man erhält 10,5 g(75 % der Theorie) 1-(4-Chlorphenyl)-3-[4-(4'-chlorphenoxy)-phenyl]-2-(1,2,4-triazol-1-yl)-propan-3-ol vom Schmelzpunkt 112°C.

In analoger Weise und entsprechend den erfindungsgemäßen Verfahren werden die in der nachfolgenden Tabelle aufgeführten Verbindungen der allgemeinen Formel (IA)

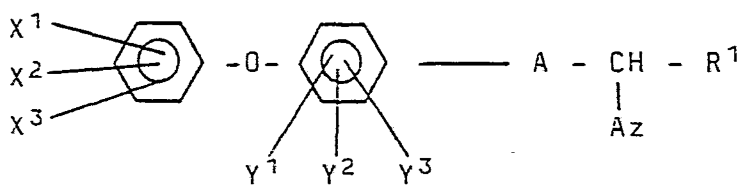 (IA)

| Bsp. Nr. | $X^1$ | $X^2$ | $X^3$ | $Y^1$ | $Y^2$ | $Y^3$ | A | Az | $R^1$ | Fp(°C) bzw. $n_D^{20}$ |
|---|---|---|---|---|---|---|---|---|---|---|
| 5 | H | H | H | H | H | H | -CO- | 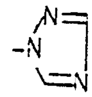 | $-C_3H_7$-n | 66-68 |
| 6 | 4-Cl | H | H | H | H | H | -CO- | 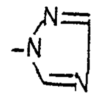 | $-C_3H_7$-n | 64-66 |
| 7 | 4-Cl | H | H | H | H | H | -CO- | 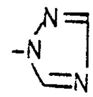 | $-C_2H_5$ | 91-92 |
| 8 | H | H | H | H | H | H | -CO- | 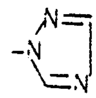 | $-C_2H_5$ | 62-63 |
| 9 | 4-Cl | H | H | H | H | H | -CO- | 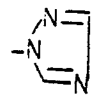 | $-CH_3$ | 1,6061 |
| 10 | 4-Cl | H | H | H | H | H | -CO- | 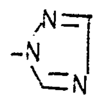 | $-C_4H_9$-n | 88 |
| 11 | 4-Cl | H | H | H | H | H | -CO- | 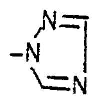 | 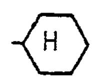 | 126 |
| 12 | 4-Cl | H | H | H | H | H | -CO- | 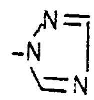 | $-C_5H_{11}$-n | 64 |
| 13 | 4-Cl | H | H | H | H | H | -CO- | 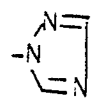 | $-C_3H_7$-i | 92-93 |
| 14 | 4-Cl | H | H | H | H | H | -CO- | 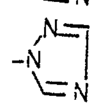 | $-CH_2C{\equiv}CH$ | 116 |
| 15 | 4-Cl | H | H | H | H | H | -CO- | 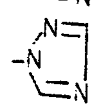 | $-CH_2CH{=}CH_2$ | 87 |
| 16 | 4-Cl | H | H | H | H | H | -CO- | 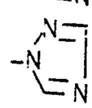 | $-C_7H_{15}$-n | 67 |
| 17 | 4-Cl | H | H | H | H | H | -CO- | 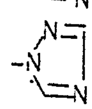 | $-CH_2CH{=}CHCH_3$ | 87 |
| 18 | 4-Cl | H | H | H | H | H | -CO- | 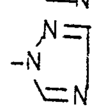 | $-CH_2CH_2OCH_3$ | 1,5940 |
| 19 | 4-Cl | H | H | H | H | H | -CO- | 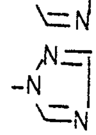 | $-CH{=}CHC_3H_7$ | 1,5840 |
| 20 | 2-Cl | 4-Cl | H | H | H | H | -CO- | 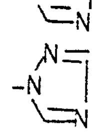 | $-C_3H_7$-n | 106 |

| Bsp. Nr. | $X^1$ | $X^2$ | $X^3$ | $Y^1$ | $Y^2$ | $Y^3$ | A | Az | $R^1$ | Fp(°C)bzw. $n_D^{20}$ |
|---|---|---|---|---|---|---|---|---|---|---|
| 21 | $2-CH_3$ | 6-Cl | H | H | H | H | -CO- | 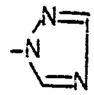 | H | 93-97 |
| 22 | 2-Cl | 4-Cl | H | H | H | H | -CO- | 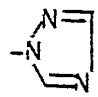 | H | 100 |
| 23 | 4-Br | H | H | H | H | H | -CO- | 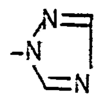 | H | 150 |
| 24 | 4-Cl | H | H | H | H | H | -CO- | 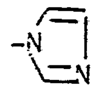 | $-C_2H_5$ | 1,6092 |
| 25 | 4-Cl | H | H | H | H | H | -CO | 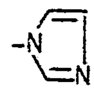 | $-C_3H_7$-n | 1,6040 |
| 26 | 4-Cl | H | H | H | H | H | -CO- | 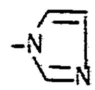 | $-CH_3$ | 1,5990 |
| 27 | 4-Cl | H | H | H | H | H | -CO- | 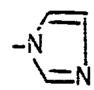 | $-C_4H_9$-n | 1,5959 |
| 28 | 4-Cl | H | H | H | H | H | -CO | 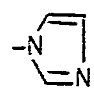 | $-C_3H_7$-i | 114-116 |
| 29 | 4-Cl | H | H | H | H | H | -CO- | 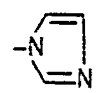 | H | 212 ($xCuCl_2$) |
| 30 | 4-Cl | H | H | H | H | H | -CH(OH)- | 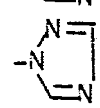 | $-C_2H_5$ | Oel(A-Form) |
| 31 | 4-Cl | H | H | H | H | H | -CH(OH)- | 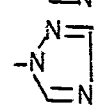 | $-C_2H_5$ | 161-62 (B-Form) |
| 32 | 4-Cl | H | H | H | H | H | -CH(OH)- | 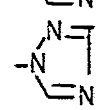 | $-C_3H_7$-n | 150(A-Form) |
| 33 | 4-Cl | H | H | H | H | H | -CH(OH)- | 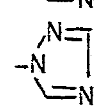 | $-C_3H_7$-n | 106-07 (B-Form) |
| 34 | 4-Cl | H | H | H | H | H | -CH(OH)- | 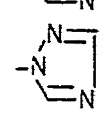 | $-C_4H_9$-n | 90(A-Form) |
| 35 | 4-Cl | H | H | H | H | H | -(CH(OH)- | 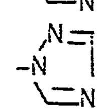 | $-C_4H_9$-n | 116 (B-Form) |
| 36 | 4-CL | H | H | H | H | H | -CH(OH)- | 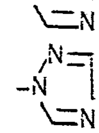 | $-CH_3$ | 90-100 |

| Bsp. Nr. | $X^1$ | $X^2$ | $X^3$ | $Y^1$ | $Y^2$ | $Y^3$ | A | Az | $R^1$ | Fp(°C)bzw. $n_D^{20}$ |
|---|---|---|---|---|---|---|---|---|---|---|
| 37 | H | H | H | H | H | H | -CH(OH)- | 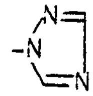 | $-CH_3$ | 1,5842 |
| 38 | H | H | H | H | H | H | -CH(OH)- | 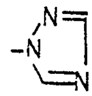 | $-C_3H_7$-n | 96(A-Form) |
| 39 | H | H | H | H | H | H | -CH(OH)- | 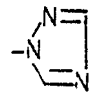 | $-C_3H_7$-n | 102 (B-Form) |
| 40 | H | H | H | H | H | H | -CH(OH)- | 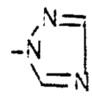 | $-C_3H_7$-i | 114-117 |
| 41 | 4-Cl | H | H | H | H | H | -CH(OH)- | 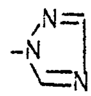 | 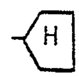 | 158 |
| 42 | H | H | H | H | H | H | -CH(OH)- | 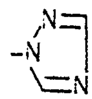 | $-C_2H_5$ | 121 (A-Form) |
| 43 | H | H | H | H | H | H | -CH(OH)- | 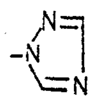 | $-C_2H_5$ | 120 (B-Form) |
| 44 | 4-Cl | H | H | H | H | H | -CH(OH)- | 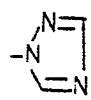 | $-C_5H_{11}$-n | 84 (A-Form) |
| 45 | 4-Cl | H | H | H | H | H | -CH(OH)- | 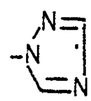 | $-C_5H_{11}$-n | 102 (B-Form) |
| 46 | 4-Cl | H | H | H | H | H | -CH(OH)- | 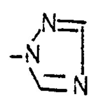 | $-C_3H_7$-i | 161-63 (A-Form) |
| 47 | 4-Cl | H | H | H | H | H | -CH(OH)- | 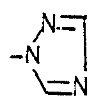 | $-C_3H_7$-i | 74-76 (B-Form) |
| 48 | 4-Cl | H | H | H | H | H | -CH(OH)- | 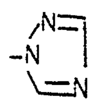 | $-C_4H_9$-i | 110-14 |
| 49 | 4-Cl | H | H | H | H | H | -CH(OH)- | 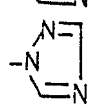 | $-C_4H_9$-i | 107 (A-Form) |
| 50 | 4-Cl | H | H | H | H | H | -CH(OH)- | 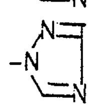 | $-C_4H_9$-i | 151 (B-Form) |
| 51 | 4-Cl | H | H | H | H | H | -CH(OH)- | 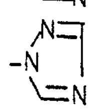 | $-C_3H_7$-i | 160-62 |
| 52 | 4-Cl | H | H | H | H | H | -CH(OH)- | 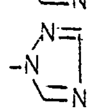 | $-CH_2C\equiv CH$ | 90 |
| 53 | 4-Cl | H | H | H | H | H | -CH(OH)- | 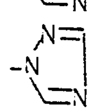 | $-CH_2CH=CH_2$ | 107 |

| Bsp. Nr. | $X^1$ | $X^2$ | $X^3$ | $Y^1$ | $Y^2$ | $Y^3$ | - A | Az | $R^1$ | Fp(°C)bzw. $n_D^{20}$ |
|---|---|---|---|---|---|---|---|---|---|---|
| 54 | 4-Cl | H | H | H | H | H | -CH(OH)- | 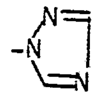 | $-C_7H_{15}$-n | 74 |
| 55 | 4-Cl | H | H | H | H | H | -CH(OH)- | 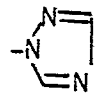 | $-CH_2CH=CHCH_3$ | 109 |
| 56 | 4-Cl | H | H | H | H | H | -CH(OH)- | 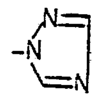 | $-CH=CHC_3H_7$ | 1,5608 |
| 57 | 4-Cl | H | H | H | H | H | -CH(OH)- | 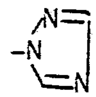 | $-CH_2CH_2OCH_3$ | 95-100 |
| 58 | 2-Cl | 4-Cl | H | H | H | H | -CH(OH)- | 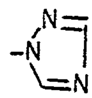 | $-C_3H_7$-n | zähes Oel |
| 59 | 2-Cl | 4-Cl | H | H | H | H | -CH(OH)- | 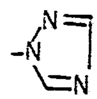 | $-CH_3$ | 200(xHCl ) |
| 60 | 4-Cl | H | H | H | H | H | -C (OH) $CH_2$- 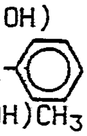 | 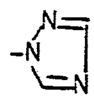 | $-C_3H_7$-n | Oel |
| 61 | 4-Cl | H | H | H | H | H | $-C(OH)CH_3$ | 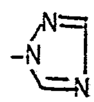 | $-C_3H_7$-n | 145 |
| 62 | 4-Cl | H | H | H | H | H | $-C(OH)CH_3$ | 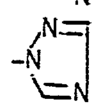 | $-CH_3$ | 128 |
| 63 | 4-Cl | H | H | H | H | H | -C(OH) $CH_2$- 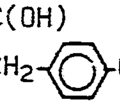 -Cl | 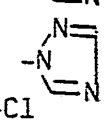 | $-CH_3$ | 142 |
| 64 | 4-Cl | H | H | H | H | H | -C(OH) $CH_2-CH=CH_2$ | 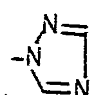 | $-C_3H_7$-n | 128 |
| 65 | $2-CH_3$ | 6-Cl | H | H | H | H | -CH(OH)- | 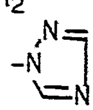 | H | 125-26 |
| 66 | 2-Cl | 4-Cl | H | H | H | H | -CH(OH)- | 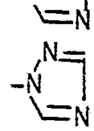 | H | zähes Oel |
| 67 | 4-Cl | H | H | H | H | H | -CH(OH)- | 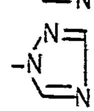 | H | 114 |
| 68 | 4-Br | H | H | H | H | H | -CH(OH)- | 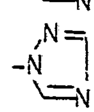 | H | 106 |

| Bsp. Nr. | $X^1$ | $X^2$ | $X^3$ | $Y^1$ | $Y^2$ | $Y^3$ - A | Az | $R^1$ | Fp(°C)bzw. $n_D^{20}$ |
|---|---|---|---|---|---|---|---|---|---|
| 69 | H | H | H | H | H | H -CH(OH)- | 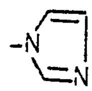 | $-C_3H_7$-n | 1,5600 |
| 70 | H | H | H | H | H | H -CH(OH)- | 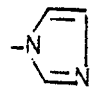 | $-C_3H_7$-i | 128 |
| 71 | 4-Cl | H | H | H | H | H -CH(OH)- | 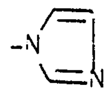 | $-C_2H_5$ | 48-52 |
| 72 | 4-Cl | H | H | H | H | H -CH(OH)- | 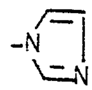 | $-CH_3$ | zähes Oel |
| 73 | 4-Cl | H | H | H | H | H -CH(OH)- | 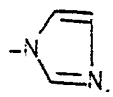 | $-C_4H_9$-n | 120-30 |
| 74 | 4-Cl | H | H | H | H | H -CH(OH)- | 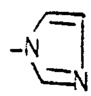 | $-C_3H_7$-i | 50-55 |
| 75 | 4-Cl | H | H | H | H | H -CH(OH)- | 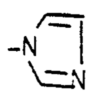 | $-C_3H_7$-n | 44-48 |

A- und B-Form= die beiden möglichen geometrischen Isomeren

In den nachfolgenden Beispielen werden die nachstehend angegebenen Verbindungen als Vergleichssubstanzen eingesetzt:

(A) = 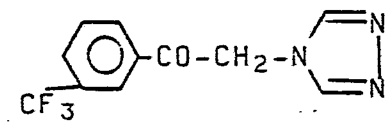

(B) = 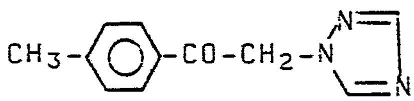

(C)= 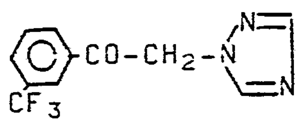

(D)= 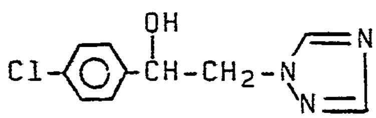

Beispiel A

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 9, 10, 41, 5, 7, 6, 8, 40 und 13.

0077479

Beispiel 8

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 69,9,10,36,41,4,37,5,7,6,8,40 und 13.

Beispiel C

Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäß folgender Herstellungsbeispiele: 36, 7 und 74.

Beispiel D

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 36, 7 und 74.

Patentansprüche

1) Phenoxyphenyl-azolylmethyl-ketone und -carbinole der allgemeinen Formel

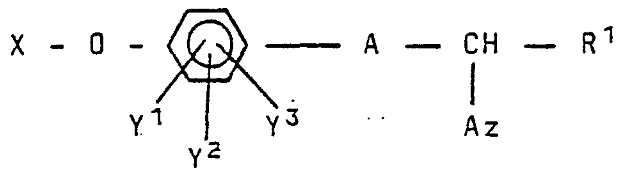
(I)

in welcher

Az für 1,2,4-Triazol-1-yl und -4-yl oder Imidazol-1-yl steht,

A für die Ketogruppe, die -CH(OH)- oder die -C(OH)R-Gruppierung steht,

R für Alkyl, Cycloalkyl, Cycloalkylalkyl, Alkenyl, Alkinyl, gegebenenfalls substituiertes Phenyl oder gegebenenfalls substituiertes Phenalkyl steht,

$R^1$ für Alkyl, Cycloalkyl, Cycloalkylalkyl, Alkenyl, Alkinyl, Halogenalkyl, Alkoxyalkyl, Halogenalkoxyalkyl, Alkylthioalkyl, Halogenalkylthioalkyl, Dialkylaminoalkyl oder gegebenenfalls substituiertes Phenalkyl steht, sowie auch für Wasserstoff steht, wenn nicht gleichzeitig X für unsubstituiertes Phenyl und $Y^1$,$Y^2$ und $Y^3$ für Wasserstoff stehen, und ferner noch für Wasserstoff steht, wenn R nicht für Alkyl, Cycloalkyl oder Cycloalkylalkyl steht,

X für gegebenenfalls einfach oder mehrfach, gleich oder verschieden substituiertes Phenyl steht,

$Y^1$, $Y^2$, $Y^3$ gleich oder verschieden sind und für Wasserstoff, Halogen oder Alkyl stehen,

sowie deren pflanzenverträglichen Säureadditions-Salze und Metallsalz-Komplexe.

2) Verbindungen der Formel I in Anspruch 1, in welcher

A und Az die in Anspruch 1 angegebene Bedeutung haben,

X für die Gruppe 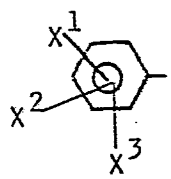 steht,

R für geradkettiges oder verzweigtes Alkyl mit 1 bis 4 Kohlenstoffatomen, Cycloalkyl und Cycloalkylmethyl mit jeweils 5 oder 6 Kohlenstoffatomen im Cycloalkylteil, Alkenyl und Alkinyl mit jeweils 2 bis 4 Kohlenstoffatomen, gegebenenfalls substituiertes Phenyl und Benzyl steht, wobei als Substituenten genannt seien: Fluor, Chlor, Brom und Methyl,

$R^1$ für geradkettiges oder verzweigtes Alkyl mit 1 bis 8 Kohlenstoffatomen, Cycloalkyl und Cycloalkylmethyl mit jeweils 5 oder 6 Kohlenstoffatomen im Cycloalkylteil, Alkenyl mit 2 bis 6 Kohlenstoffatomen, Alkinyl mit 2 bis 4 Kohlenstoffatomen, Alkoxyalkyl und Alkylthioalkyl mit jeweils 1 bis 2 Kohlenstoffatomen in jedem Alkylteil, Halogenalkyl mit 1 bis 4 Kohlenstoff- und 1 bis 5 Halogenatomen, wie insbesondere Fluor- und Chloratomen, Halogenalkoxyalkyl und Halogenalkylthioalkyl mit jeweils 1 bis 2 Kohlenstoffatomen in jedem Alkylteil und 1 bis 5 Halogenatomen, wie insbesondere Fluor- und Chloratomen, Dialkylaminoalkyl mit 1 bis 2 Kohlenstoffatomen in jedem Alkylteil, und gegebenenfalls substituiertes Benzyl steht, wobei als Phenylsubstituenten die für $X^1$ genannten Reste infrage kommen, sowie auch für Wasserstoff steht, wenn nicht gleichzeitig $X^1$, $X^2$, $X^3$, $Y^1$, $Y^2$ und $Y^3$ für Wasserstoff stehen, und ferner noch für Wasserstoff steht, wenn R nicht für Alkyl, Cycloalkyl oder Cycloalkylalkyl steht,

$X^1$ für Wasserstoff, Fluor, Chlor,Brom, Methyl, Trifluormethyl, Trifluormethoxy, Trifluormethylthio, Methoxy, Methylthio, Amino, Dimethylamino, Methoxycarbonyl, Nitro oder Cyano steht,

$X^2$ für Wasserstoff, Fluor, Chlor, Methyl oder Methoxy steht, oder

$X^1$ und $X^2$ gemeinsam in o-Stellung zueinander für Methylendioxo stehen,

$X^3$ für Wasserstoff, Fluor, Chlor, Methyl oder Methoxy steht,

$Y^1$ für Wasserstoff, Fluor, Chlor oder Methyl steht,

$Y^2$ für Wasserstoff, Fluor oder Chlor steht und

$Y^3$ für Wasserstoff steht.

3) Verfahren zur Herstellung von Phenoxyphenyl-azolylmethyl-ketonen und -carbinolen der allgemeinen Formel

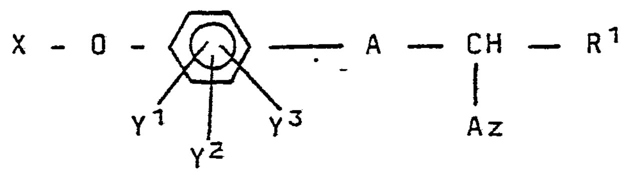

(I)

in welcher

Az für 1,2,4-Triazol-1-yl und -4-yl oder Imidazol-1-yl steht,

A für die Ketogruppe, die -CH(OH)- oder die -C(OH)R-Gruppierung steht,

R für Alkyl, Cycloalkyl, Cycloalkylalkyl, Alkenyl, Alkinyl, gegebenenfalls substituiertes Phenyl oder gegebenenfalls substituiertes Phenalkyl steht,

$R^1$ für Alkyl, Cycloalkyl, Cycloalkylalkyl, Alkenyl, Alkinyl, Halogenalkyl, Alkoxyalkyl, Halogenalkoxyalkyl, Alkylthioalkyl, Halogenalkylthioalkyl, Dialkylaminoalkyl oder gegebenenfalls substituiertes Phenalkyl steht, sowie auch für Wasserstoff steht, wenn nicht gleichzeitig X für unsubstituiertes Phenyl und $Y^1$,$Y^2$ und $Y^3$ für Wasserstoff stehen, und ferner noch für Wasserstoff steht, wenn R nicht für Alkyl, Cycloalkyl oder Cycloalkylalkyl steht,

X für gegebenenfalls einfach oder mehrfach, gleich oder verschieden substituiertes Phenyl steht,

$Y^1$, $Y^2$, $Y^3$ gleich oder verschieden sind und für Wasserstoff, Halogen oder Alkyl stehen,

dadurch gekennzeichnet, daß man

a) Halogenketone der Formel

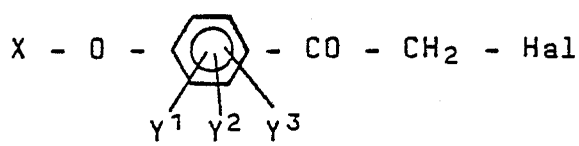 (II)

in welcher

Hal für Halogen, vorzugsweise Chlor oder Brom steht und

X, $Y^1$, $Y^2$ u. $Y^3$ die oben angegebene Bedeutung haben,

mit Azolen der Formel

H - Az (III)

in welcher

Az die oben angegebene Bedeutung hat,

in Gegenwart eines Verdünnungsmittels und in Gegenwart eines Säurebindemittels umsetzt; und gegebenenfalls

b) die so erhaltenen Azolylketone der Formel

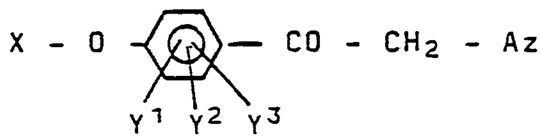 (Ia)

in welcher

Az, X, $Y^1$, $Y^2$ und $Y^3$ die oben angegebene Bedeutung haben,

mit einem Alkylierungsmittel der Formel

$R^1$ - Z (IV)

in welcher

$R^1$ die oben angegebene Bedeutung hat und

Z für eine elektronenanziehende Abgangsgruppierung steht,

in Gegenwart einer Base und in Gegenwart eines organischen Verdünnungsmittels, oder in einem wässrig-organischen Zweiphasensystem in Gegenwart eines Phasentransfer-Katalysators, umsetzt; und gegebenenfalls

c) die nach den Verfahren (a) und (b) erhaltenen Azolylketone der Formel

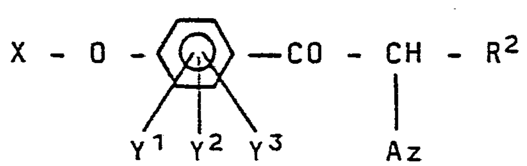 (Ib)

in welcher

$Az, X, Y^1, Y^2$ und $Y^3$ die oben angegebene Bedeutung haben und

$R^2$ für Wasserstoff oder $R^1$ steht, wobei $R^1$ die oben angegebene Bedeutung hat,

durch Reaktion mit komplexen Hydriden bzw. Aluminiumisopropylat in Gegenwart eines Verdünnungsmittels reduziert oder mit metallorganischen Verbindungen der Formel

Me - R (V)

in welcher

R die oben angegebene Bedeutung hat und

Me für ein Alkalimetall oder den Rest Hal'-Mg steht, wobei

Hal' für Chlor, Brom oder Iod steht,

in Gegenwart eines Verdünnungsmittels umsetzt. und schließlich gegebenenfalls an die so erhaltenen Verbindungen der Formel (I) noch anschließend eine Säure oder ein Metallsalz addiert.

4) Fungizide Mittel, gekennzeichnet durch einen Gehalt an mindestens einem Phenoxyphenyl-azolylmethyl-keton oder -carbinol der Formel I.

5) Verfahren zur Bekämpfung von Pilzen, dadurch gekennzeichnet, daß man Phenoxyphenyl-azolylmethyl-ketone oder -carbinole der Formel I auf Pilze oder ihren Lebensraum einwirken läßt.

6) Verwendung von Phenoxyphenyl-azolylmethyl-ketonen oder -carbinolen der Formel I zur Bekämpfung von Pilzen.

7) Verfahren zur Herstellung von fungiziden Mitteln, dadurch gekennzeichnet, daß man Phenoxyphenyl-azolylmethyl-ketone oder -carbinole der Formel I mit Streckmitteln und/oder oberflächenaktiven Mitteln vermischt.

8) Verwendung von Phenoxyphenyl-azolylmethyl-ketonen oder -carbinolen der Formel I als Zwischenprodukte zur Herstellung von Pflanzenschutzmitteln.